# EUROPEAN PATENT APPLICATION

(11) **EP 3 578 141 A1**
(43) Date of publication of application: **11.12.2019**
(21) Application number: 18181993.9
(22) Date of filing: 05.07.2018
(51) Int. Cl.: A61F 2/90

(54) **MEDICAL STENT DEVICE**

(30) Priority: 07.06.2018 KR 20180065592
(71) Applicant: First Medical Co., Ltd., Tokyo 133-0051 (JP); Go, Moon-hwa, Cheonan-si 31119 (KR)
(72) Inventor: Kim, Ju-yul, Tokyo, 133-0051 (JP); Go, Moon-hwa, 31119 Chungcheongnam-do (KR)
(74) Representative: Schön, Christoph

(57) **Abstract**

Provided is a medical stent device in which a first woven portion and a second woven portion are arranged alternately in an axial direction. The first woven portion is configured to have a mesh structure including a plurality of rhombic meshes and is formed by weaving a plurality of wires such that each of the wires crosses one another to form crests and valleys. The second woven portion is elongated in the axial direction from the first woven portion. In the second woven portion, a traveling direction of each of the wires extending from the first woven portion is bent into the axial direction.

## Description

### CROSS-REFERENCE TO RELATED APPLICATION

This application claims the benefit of Korean Patent Application No. 10-2018-0065592, filed on June 7, 2018, in the Korean Intellectual Property Office, the disclosure of which is incorporated herein in its entirety by reference.

### BACKGROUND

### 1. Field

The present disclosure relates to a stent and, more particularly, to a medical stent to be inserted into a lumen of a human body such as an esophagus and a blood vessel for securing a passage.

### 2. Description of the Related Art

When a stenosis occurs in a lumen of a human body such as esophagus, duodenum, bile duct, urethra, or airway due to a tumor or other cause, the organ cannot function normally. In such a situation, a stent is inserted into the stenosis site to expand the lumen. In other words, the stent is widely being used to keep the passageway of the human lumen open.

The stent is composed of a hollow cylindrical body having a plurality of rhombic meshes formed by interweaving wires of a hyperelastic shape memory alloy such that the wires cross in a diagonal direction. Such a hollow cylindrical body exerts a radial tension against the lumen wall to expand the stenosis site.

Since the stent include a plurality of rhombic meshes formed by interweaving the wires, a strain applied in one side of the stent may induce an elastic movement of the stent in the axial or longitudinal direction, which may result in a migration of the stent from its original position. In such a case, the stent cannot relieve the stenosis at the lesion site, and the patient may have to be subject to a new stenting procedure.

In order to prevent the migration of the stent, Korean Patent No. 10-1657648 entitled MANUFACTURING METHODS FOR ANTI-MIGRATION STENT AND THE ANTI-MIGRATION STENT THEREOF disclose a stent in which a shoulder bump protruding in a radial direction is formed to prevent a migration of the stent. This stent disclosed in this document, however, may have disadvantages that it is difficult to maintain a shape of the shoulder bump for a long time, the shoulder bump may lower a lateral compression ratio. Further, the stent may exhibit a large axial expansion ratio enough to result in the migration of the stent from the lesion site to another position after the procedure.

Korean Unexamined Patent Publication No. 2013-0126776 entitled MEDICAL STENT HAVING ENDS WITH RADIAL FORCE THAN CENTRAL PORTION discloses a stent in which the wires are loosely twisted near the ends of the stent than its central portion so that a radial force in end portions is smaller than the central portion and a pressure applied to a certain position on the surface is prevented from being propagated in the axial direction. However, the stent described in this document may have a low lateral compression ratio, and it is difficult to laterally compress the stent during the procedure, and thus it is difficult to insert the stent into the narrow lumen of the human body. In addition, since the wires are bent in the opposite direction, it is difficult to automate the manufacture of the stent, which may result in a low production yield and a high production cost of the stent.

### SUMMARY

In order to solve the problems above, provided is a stent capable of preventing a strain occurred in a certain position on the stent from being propagated along an axial direction and having a structure that maximizes a lateral compression ratio so as to be inserted easily into a narrow lumen of a human body.

According to an embodiment of the present disclosure, a stent device includes a first woven portion and a second woven portion. The first woven portion is configured to have a mesh structure including a plurality of rhombic meshes and is formed by weaving a plurality of wires such that each of the wires crosses one another to form crests and valleys. The second woven portion is elongated in an axial direction from the first woven portion. In the second woven portion, a traveling direction of each of the wires extending from the first woven portion is bent into the axial direction. The second woven portion may have a cylindrical shape. The first woven portion and the second woven portion are arranged alternately in the axial direction.

In the second woven portion, two adjacent wires may be weaved in such a manner that the two adjacent wires cross each other in a twisted fashion.

In the second woven portion, the two adjacent wires may cross each other twice or more times.

The first woven portion may be longer than the second woven portion.

The first woven portion may be shorter than the second woven portion.

According to the stent of the present disclosure, the second woven portion has a bent portion where the wires are bent in the axial direction, and a strain occurred in a certain position on the stent is prevented from being propagated along the axial direction.

A weaving pattern of adjacent wires in a twisted fashion according to the present disclosure exhibits a lateral compression ratio which is comparable to that of a mesh structure including a plurality of rhombic meshes and is a than a weaving pattern of a conventional hook structure, which facilitates the insertion of the stent into a narrow lumen of a human body.

According to the stent of the present disclosure, the compression ratio can be changed only by adjusting relative lengths of the first and second woven portions, and thus the stent may be adapted easily for the stenting site.

Also, according to the stent of the present disclosure, it is possible to automate the manufacture of the stent contrary to a conventional stent having a hook structure, which may enhance a production yield and lower a production cost of the stent.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1A is a photograph of a stent according to an embodiment of the present disclosure;
FIG. 1B is a development view of a stent according to an embodiment of the present disclosure;
FIG. 2 illustrates operational characteristics of a stent according to an embodiment of the present disclosure;
FIG. 3 is a development view of a stent according to another embodiment of the present disclosure; and
FIG. 4 is a development view of a stent according to yet another embodiment of the present disclosure.

### DETAILED DESCRIPTION

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects.

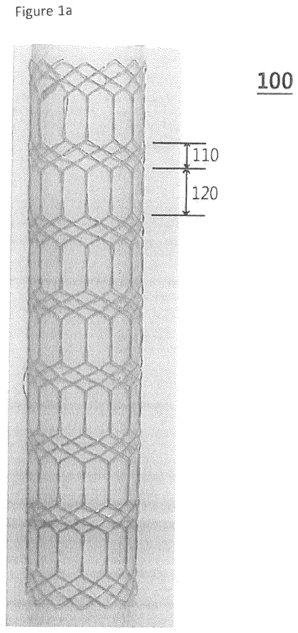

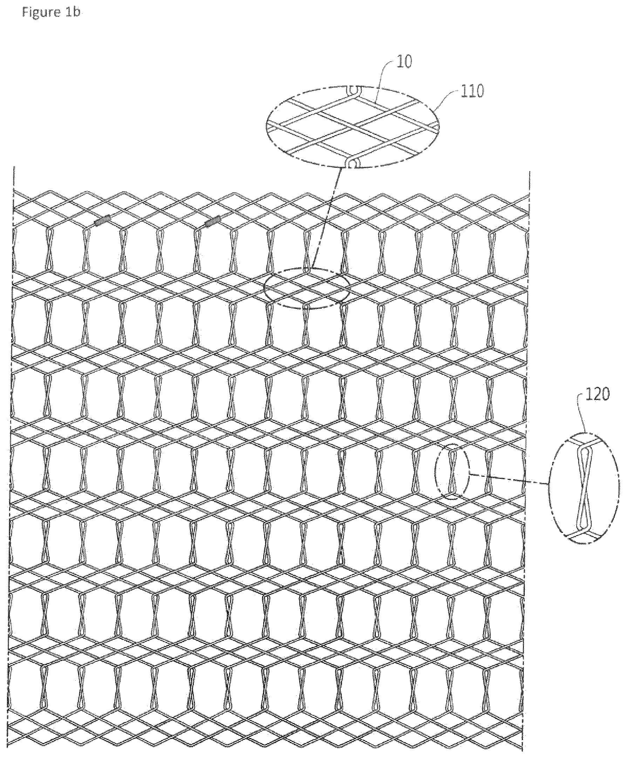

FIG. 1A is a photograph of a stent according to an embodiment of the present disclosure, and FIG. 1B is a development view of the stent.

As shown in FIGS. 1A and 1B, the stent 100 according to an embodiment of the present disclosure may include two woven portions 110 and 120 which are formed by weaving a plurality of wires 10 and have different shapes.

The wire 10 may be made of a elastic material such as a metal, a synthetic polymer, a natural polymer, or the like.

Preferably, the metal may be a shape memory alloy such as a nickel-titanium (Ni-Ti) shape memory alloy and a martensitic Ni-Ti shape memory alloy. However, the kind of metal is not limited thereto, and the metal may be stainless steel, tantalum, tungsten, gold, platinum, silver, nickel, titanium, chromium, a cobalt-chrome (Co-Cr) alloy, a platinum-chrome (Pt-Cr) alloy, a platinum-iridium (Pt-Ir) alloy, and a magnesium alloy.

The synthetic polymer may be one of degradable polymers and non-degradable polymers. Examples of the degradable polymer include poly(lactic acid) and its copolymers, poly(glycolic acid) and its copolymers, (poly(hydroxy butyrate), poly(e-caprolactone) and its copolymers, poly(alkylene succinates), polyanhydrides, and poly(ortho esters). Examples of the non-degradable polymer include polyamides, poly(cyano acrylates), polyphosphazenes, thermoplastic polyurethanes, low density polyethylene, poly(vinyl alcohol), poly(ethylene oxide), poly(hydroxyethyl methacrylate), poly(methyl methacrylate), poly(tetrafluoroethylene) (PTFE), polydimethylsiloxane, poly(ethylene oxide-b-propylene oxide), poly(vinyl methyl ether), poly(N-alkyl acrylamide), and polyethylene terephthalate, polypropylene.

Examples of the natural polymers include collagen, albumin, silk protein, poly(L-lysine), (poly(L-glutamic acid), polyaspartic acid, polysaccharide and derivatives thereof, carboxymethylcellulose, cellulose sulfate, agarose, alginate, carrageenan, hyaluronic acid, heparin, glycosaminoglycan, dextran and derivatives thereof, and chitosan and derivatives thereof.

The wire 10 may be made of one material in order to keep an axial expansion ratio and a lateral compression ratio uniform. However, the present disclosure is not limited thereto, and the wire 10 may be formed by combining two or more materials.

The wire 10 can be coated so as to be used in a lumen of a human body.

The stent 100 may include a first woven portion 110, and a second woven portion 120. The first and second woven portions 110 and 120 may be alternately arranged in the axial direction.

The first woven portion 110 has a mesh structure including a plurality of rhombic meshes and may be formed by weaving a plurality of wires 10 such that each of the wires 10 crosses one another to form crests and valleys. In the first woven portion 110, the plurality of wires 10 extend in the axial direction but are arranged obliquely at a certain inclination angle to form a spiral shape spatially. In the first woven portion 110, the inclination angle of the wires 10 may be chosen in a range of 20-70 degrees when being measured with reference to an imaginary vertical line while the stent is rotated. The inclination angle may be chosen in accordance with the diameter of the lumen of the human body, and the axial expansion ratio and the lateral compression ratio are getting larger as the inclination angle increases. For example, when a diameter of the stenosis site is small, the helical inclination angle of the wires 10 may be chosen to be a small value. As the diameter of the stenosis site is larger, the helical inclination angle of the wires 10 may be chosen to be a larger value.

In the first woven portion 110, each wire may be extended continuously without changing its traveling direction (i.e., without being bended to another direction) while maintaining the inclination angle.

The first woven portion 110 may be formed by weaving directly into a hollow cylindrical shape. Instead, however, the first woven portion 110 may be bent into the hollow circular shape after weaving in a flat development shape.

As described above, the first woven portion 110 has the mesh structure having a plurality of rhombic meshes, is capable of maintaining a maximum an axial expansion ratio and lateral compression ratio since the traveling direction of the wire 10 is extended without being changed.

The second woven portion 120 has a cylindrical shape and extends in the axial direction. The second woven portion 120, which is provided to block or minimize the axial expansion of the stent 100, may be formed by bending the wires into the downward direction.

In detail, the second woven portion 120 may be formed by bending each of the wires 10 downwards at an end of the first woven portion 110, so that the travelling direction of the wires are changed into the axial direction. Accordingly, the traveling direction of the wires 10 changes from the helical shape into an almost vertically straight-line shape disposed in the axial direction.

When the inclination angle of the wires 10, which is measured with reference to an imaginary vertical line while the stent is rotated, is zero, the wires 10 would be disposed in the axial direction. Contrarily, when the inclination angle of the wires 10 is greater than zero degree, the wires 10 would have the helical shape. In case the wires 10 have the helical shape, the axial expansion may occur in the second woven portion 120 also. If the wires 10 are provided with an inclination angle other than zero degree, the inclination angle may be chosen from a range from 0 to 20 degrees in consideration that the inclination angle of the wires in the first woven portion 110 ranges from 20 to 70 degrees.

The second woven portion 120 can be constructed by weaving two adjacent wires 10, of which traveling directions are changed into the axial direction, such that the two adjacent wires 10 cross each other in a twisted fashion. The second woven portion 120 may have a multiple crossing structure that the adjacent wires 10 cross each other twice or more times. If the adjacent wires 10 cross each other just a single time, the travelling direction of each of the wires 10 will be reversed when the second woven portion 120 is terminated and the first woven portion 110 starts again. If the adjacent wires 10 cross each other twice, the travelling direction of each of the wires 10 will remain unchanged when the second woven portion 120 is terminated and the first woven portion 110 starts again. Similarly, the travelling direction of each of the wires 10 will be reversed if the wires 10 cross each other an odd number of times, but will remain unchanged if the wires 10 cross each other an even number of times. In case that it is necessary to keep the axial expansion ratio and the lateral compression ratio of the first woven portion 110 at respective maximum levels, it is preferable that the adjacent wires 10 of the first woven portions 110 disposed in both ends of the second woven portion 120 travel in the same direction as each other. Thus, in such a case, it is preferable that the crossing of the adjacent wires 10 in the second woven portion 120 is provided an even number of times.

When the second woven portion 120 is disposed in the axial direction, the axial expansion ratio is almost zero. Thus, the longer the second woven portion 120 is, the smaller the axial expansion ratio of the entire stent 100 is. Contrarily, if the second woven portion 120 is shortened, the axial expansion ratio of the entire stent 100 is increased.

Though the wires 10 are bent in the second woven portion 120, the traveling directions of the wires 10 remain unchanged unlike a hook structure in which the traveling directions of the wires 10 are symmetrically reversed. Thus, the lateral compression ratio of the second woven portion 120 can be maintained at substantially the same level as that of the first woven portion 110 having the mesh structure.

As described above, the second woven portion 120 enables to adjust the axial expansion ratio of the stunt 100 according to a change of the length of the second woven portion 120 while the axial compression ratio of the stent 100 is maintained almost constant. As a result, the stunt 100 can be implanted into a narrow lumen of the human body such as a blood vessel with no problem, and a strain applied in one side of the stent 100 can be blocked from being propagated to adjacent portions and the migration of the stent 100 due to the peristalsis of the lumen of the human body can be effectively prevented.

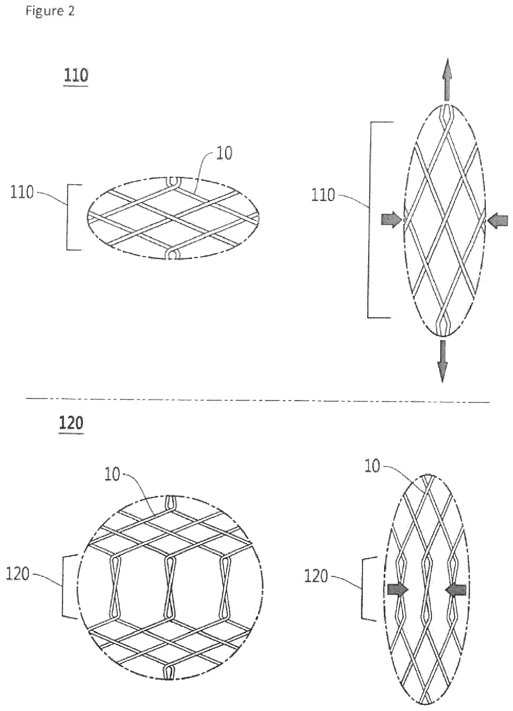

FIG. 2 illustrates operational characteristics of the stent 100 according to an embodiment of the present disclosure.

As shown in FIG. 2, the first woven portion 110 can be stretched in the axial direction while being compressed in the lateral direction when a lateral pressure is applied.

Contrarily, when the lateral pressure is applied, the second woven portion 120 is compressed in the lateral direction but shows little change in the axial direction. In particular, when the wires 10 of the second woven portion 120 are elongated in the axial direction as shown in FIG. 2, there is little expansion of the second woven portion 120 in the axial direction.

There are several evaluation indexes for evaluating the characteristics of the stent 100. In relation to an axial deformation, such evaluation indexes include shortening and anti-migration.

The shortening indicates a degree of the axial deformation due to the lateral compression. When the stent 100 having a certain diameter is compressed and mounted on a delivery device having a smaller diameter, the stent 100 is mounted in a shape of being longer than its original length, and thus the stent 100 may not be mounted correctly in an implantation target location. Therefore, it is preferable that the stent 100 shows less shortening characteristics, that is, has a smaller axial expansion ratio as long as the lateral compression ratio is sufficient.

The anti-migration indicates a degree of a displacement of a mounted stent 100 due to some reason such as the peristalsis of the lumen of the human body. Generally, higher anti-migration characteristic is beneficial to the patient.

Accordingly, conventional stents have adopted the hook structure in which the traveling directions of the wires 10 are symmetrically reversed at each joint because of its superior shortening and anti-migration characteristics. However, despite of it good shortening and anti-migration characteristics, the hook structure is disadvantageous in that the lateral compression ratio is so small that it is difficult to reduce a volume of the stent and to mount the stent on the delivery device having a smaller diameter.

However, according to the stent 100 of the present disclosure, the lateral compression ratio of the second woven portion 120 is as good as the lateral compression ratio of the first woven portion 110 as shown in FIG. 2. Thus, the stent 100 can exhibit a maximized lateral compression ratio while maintaining good shortening and anti-migration characteristics. As a result, the difficulty in mounting the stent 100 to the delivery device can be eliminated.

In addition, if the second woven portion 120 is configured to be longer than the first woven portion 110, desired shortening and anti-movement characteristics can be attained easily.

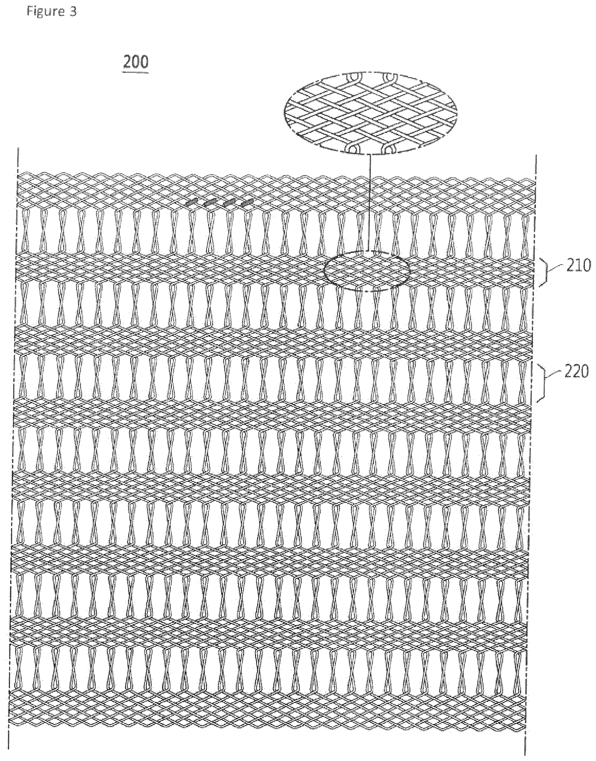

FIG. 3 is a development view of a stent according to another embodiment of the present disclosure.

In the stent 200 shown in FIG. 3, a first woven portion 210 may be configured to be longer than the second woven portion 220 or to have a length comparable to that of the second woven portion 220. According to the present embodiment, the lateral compression ratio may be increased.

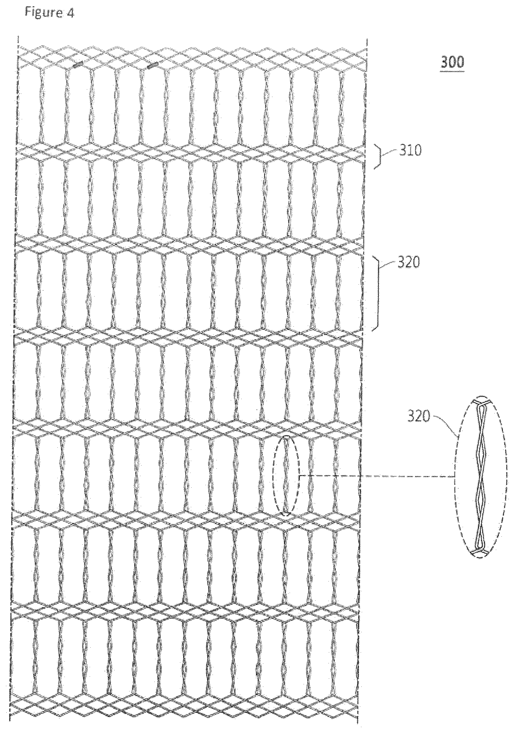

FIG. 4 is a development view of a stent according to yet another embodiment of the present disclosure.

In the stent 300 shown in FIG. 4, a first woven portion 310 may be configured to be shorter than the second woven portion 320. In this case, the axial expansion ratio of the stent 300 can be lowered while the shortening and anti-migration characteristics may be enhanced further.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the spirit and scope as defined by the following claims.

## Claims

1. A stent device, comprising:
a first cylindrical woven portion configured to have a mesh structure including a plurality of rhombic meshes and formed by weaving a plurality of wires such that each of the wires crosses one another to form crests and valleys; and
a second cylindrical woven portion elongated in an axial direction from the first cylindrical woven portion, wherein a traveling direction of each of the wires extending from the first cylindrical woven portion is bent into the axial direction,
wherein the first woven portion and the second woven portion are arranged alternately in the axial direction.

2. The stent device as claimed in claim 1, wherein, in the second cylindrical woven portion, two adjacent wires are weaved in such a manner that the two adjacent wires cross each other in a twisted fashion.

3. The stent device as claimed in claim 2, wherein, in the second cylindrical woven portion, the two adjacent wires cross each other twice or more times.

4. The stent device as claimed in any of claims 1-3, wherein the first cylindrical woven portion is longer than the second cylindrical woven portion.

5. The stent device as claimed in any of claims 1-3, wherein the first cylindrical woven portion is shorter than the second cylindrical woven portion.
